# EUROPÄISCHE PATENTSCHRIFT

(11) **EP 3 202 378 B1**
(45) Veröffentlichungstag und Bekanntmachung des Hinweises auf die Patenterteilung: **17.10.2018**
(21) Anmeldenummer: 17154790.4
(22) Anmeldetag: 06.02.2017
(51) Int. Cl.: A61F 5/01

(54) **ORTHESENGELENK UND ORTHESE**
ORTHOTIC JOINT AND ORTHOTIC
ARTICULATION ORTHOTIQUE ET ORTHÈSE

(30) Priorität: 05.02.2016 DE 102016201793
(43) Veröffentlichungstag der Anmeldung: 09.08.2017
(73) Patentinhaber: Pohlig GmbH, 83278 Traunstein (DE)
(72) Erfinder: Schäfer, Michael, 83278 Traunstein (DE); Kienzle, Christian, 83064 Raubling (DE)
(74) Vertreter: Pfenning, Meinig & Partner mbB

(56) Entgegenhaltungen:
- EP-A1- 0 362 049
- DE-A1- 19 928 269
- DE-A1-102009 013 500
- DE-A1-102012 002 552
- DE-T2- 69 434 390
- DE-U1- 20 201 352
- DE-U1-202004 020 446
- US-B2- 8 657 769

## Beschreibung

Die vorliegende Erfindung betrifft ein Orthesengelenk sowie eine Orthese zur Korrektur von Gelenkfehlstellungen sowie Lagerung und Stabilisation eines Gelenks mit einem derartigen Orthesengelenk.

Orthesen weisen üblicherweise ein erstes und ein zweites Schalenteil sowie ein Orthesengelenk auf. Die Schalenteile dienen zur Aufnahme von Körperteilen, welche durch ein körperliches Gelenk miteinander verbunden sind. Durch das Tragen einer Orthese wird der Bewegungsbereich des betreffenden Gelenks auf einen fest vorgegebenen Bewegungsbereich eingeschränkt, so dass eine Bewegung des Gelenks nur entlang vorgegebener Freiheitsgrade ermöglicht wird. Orthesen werden sowohl zur konservativen Therapie eines Gelenks als auch nach Gelenkoperationen eingesetzt, um postoperative Gelenkeinsteifungen oder einen Rückfall in eine bereits durch Operation korrigierte Gelenkfehlstellung zu vermeiden.

Die im Stand der Technik bekannten Orthesen weisen oftmals viele und große Gelenksbestandteile auf, welche das Gesamtgewicht der Orthese stark erhöhen und die allgemeine Optik der Orthese beeinträchtigen.

Die DE 10 2012 002 552 A1 offenbart eine Orthese mit einem sehr kompakten und stabilen Orthesengelenk. Gleichzeitig bietet das Orthesengelenk eine verbesserte Funktionalität, da ein größerer Bewegungsspielraum und eine Feineinstellung des Bewegungsbereichs ermöglicht werden. Aufgrund der Kompaktheit des Orthesengelenks weist die Orthese ein geringes Gewicht und eine ansprechende Optik im Verhältnis zu den vorbekannten Orthesen auf.

Problematisch bei dieser wie bei vielen anderen Orthesen ist jedoch, dass die Schalenteile der Orthese beispielsweise aus Stabilitätsgründen, optischen Gründen oder herstellungsbedingt nur wenige Verschlüsse zum Öffnen und Schließen der Orthese beim An- und Ablegen aufweisen. Das An- und Ablegen der Orthese wird hierdurch erschwert.

Die Druckschrift DE 694 34 390 T2 beschreibt ein Übungsgerät mit einem Steuermodul. Um kontrollierte Ausmaße an Widerstand gegen Bewegung in einem derartigen Übungsgeräten oder einer orthetischen Vorrichtung zu erhalten, weist das Steuermodul zusammenwirkende Widerstandselemente auf. Die Kraft zwischen den Elementen variiert in Übereinstimmung mit der Position der Elemente in Bezug zueinander. Zum Beispiel kann das Steuermodul zwei Schienen einer Knieorthese verbinden, so dass der Widerstand gegen Beugung und Streckung in Übereinstimmung mit der Position des Beines und des Oberschenkels in Bezug zueinander programmiert wird.

Ausgehend von den im Stand der Technik bekannten Problemen ist es daher eine Aufgabe der vorliegenden Erfindung ein Orthesengelenk sowie eine Orthese bereitzustellen, welche ein einfaches An- und Ablegen ermöglichen, ohne die Stabilität der Orthese zu verringern und das Gesamtgewicht zu erhöhen.

Diese Aufgabe wird durch ein teilbares Orthesengelenk nach Anspruch 1 sowie durch eine Orthese nach Anspruch 19 gelöst. Vorteilhafte Weiterbildungen des erfindungsgemäßen Orthesengelenks und der erfindungsgemäßen Orthese sind in den abhängigen Ansprüchen beschrieben.

Das erfindungsgemäße teilbare Orthesengelenk für eine bewegliche Verbindung zweier Schalenteile einer Orthese weist einen ersten Gelenkteil, der an einem ersten Schalenteil fixierbar ist, und einen relativ zum ersten Gelenkteil um eine Gelenkachse des Orthesengelenks beweglichen zweiten Gelenkteil, der an einem zweiten Schalenteil fixierbar ist, auf. Das Orthesengelenk zeichnet sich ferner durch eine Verschlussvorrichtung zum werkzeugfreien Trennen und Zusammenfügen des ersten und zweiten Gelenkteils aus.

Durch das erfindungsgemäße teilbare Orthesengelenk wird ein einfaches und werkzeugfreies Öffnen und Verschließen der Orthese direkt im Orthesengelenk und dadurch ein einfaches und rasches An- und Ablegen der Orthese ermöglicht, ohne dass die Stabilität der Orthese beeinträchtigt wird.

Vorzugsweise ist die Verschlussvorrichtung zum werkzeugfreien Trennen und Zusammenfügen des ersten und zweiten Gelenkteils an der Gelenkachse ausgebildet. Hierdurch ist ein Öffnen und Schließen der Orthese direkt im Drehpunkt bzw. auf der Gelenkachse möglich. Außerdem ist die Verschlussvorrichtung möglichst klein und unauffällig an der Orthese befestigt.

In einer besonders bevorzugten Ausgestaltung der Erfindung umfasst die Verschlusseinrichtung eine Klemmvorrichtung. Hierzu kann die Verschlussvorrichtung ein mit dem ersten Gelenkteil verbundenes Klemmelement und ein am zweiten Gelenkteil befestigtes Gegenklemmelement aufweisen, sodass zum Zusammenfügen des ersten und zweiten Gelenkteils das Klemmelement mit dem Gegenklemmelement in Richtung der Gelenkachse verklemmbar ist.

Zum Zusammenfügen des ersten und zweiten Gelenkteils ist dabei vorzugsweise der erste Gelenkteil zwischen dem Klemmelement und dem Gegenklemmelement positionierbar und der zweite Gelenkteil auf einer dem ersten Gelenkteil abgewandten Seite des Gegenklemmelements positionierbar, bezogen auf eine Richtung der Gelenkachse.

In einer besonders bevorzugten Ausgestaltung der Erfindung weist das Klemmelement ein Grundelement, eine in dem Grundelement angeordnete, senkrecht zur Gelenkachse verlaufende Bügelachse und einen an der Bügelachse drehbar aufgehängten Klemmbügel auf, wobei der Klemmbügel durch Drehen um die Bügelachse mit dem Gegenklemmelement verklemmbar und lösbar ist. Hierbei verläuft die Bügelachse vorzugsweise durch die Gelenkachse und kann insbesondere mittels einer in der Gelenkachse angeordneten Schraube oder Madenschraube gesichert sein.

Weiterhin kann der Klemmbügel vorzugsweise zwei gabelartige Enden aufweisen und an jeweils einem ersten Ast der gabelartigen Enden drehbar an der Bügelachse aufgehängt und mit jeweils einem zweiten Ast der gabelartigen Enden mit dem Gegenklemmelement verklemmbar sein. Zusätzlich kann das Gegenklemmelement zwei gegenüberliegend angeordnete Klemmnasen zum Verklemmen mit jeweils dem zweiten Ast der gabelartigen Enden des Klemmbügels aufweisen.

In einer weiteren vorteilhaften Ausgestaltung der Erfindung umfasst das Orthesengelenk ein Drehlager, das einen fest mit der Gelenkachse verbundenen Ring aufweist, auf dem der erste Gelenkteil drehbar gelagert ist. Dieser Ring kann eine ebene Mantelfläche aufweisen, so dass das Orthesengelenk ein Scharniergelenk ist. Dieser Ring weist vorzugsweise ein Material mit einem geringen Reibungskoeffizienten, insbesondere Teflon, Messing, Lagerbronze, Peek und/oder Polyurethan auf oder besteht daraus.

In einer weiteren vorteilhaften Ausgestaltung der Erfindung weist das Orthesengelenk in Richtung der Gelenkachse eine erste Zwischenscheibe zwischen dem Klemmelement und dem Ring und eine zweite Zwischenscheibe zwischen dem Ring und dem Gegenklemmelement auf. Diese Zwischenscheiben weisen bevorzugt ein Material mit einem geringen Reibungskoeffizienten, insbesondere Teflon, Messing, Lagerbronze, Peek und/oder Polyurethan auf oder bestehen daraus.

Besonders bevorzugt ist es, wenn zusätzlich zum ersten Gelenkteil die erste und die zweite Zwischenscheibe auf dem Ring gelagert sind, wobei der Ring eine Dicke aufweist, die im Wesentlichen einer Gesamtdicke der Zwischenscheiben und dem ersten Gelenkteil entspricht. In diesem Fall wirkt im geschlossenen Zustand des Orthesengelenks keine Kraft der Verschlussvorrichtung auf die Zwischenscheiben, wodurch eine Beweglichkeit des Orthesengelenks verbessert wird.

In einer weiteren vorteilhaften Ausgestaltung der Erfindung ist die Gelenkachse bis auf eine Drehbeweglichkeit des ersten Gelenkteils relativ zur Gelenkachse fest mit dem ersten Gelenkteil verbunden. Des Weiteren kann das Gegenklemmelement eine Öffnung zum Aufnehmen der Gelenkachse, beispielsweise beim Zusammenfügen, aufweisen. Besonders bevorzugt ist es, wenn die Gelenkachse einen eckigen, insbesondere dreieckigen, viereckigen, fünfeckigen, sechseckigen oder achteckigen Querschnitt aufweist und die Öffnung eine entsprechend eckige, insbesondere dreieckige, viereckige, fünfeckige, sechseckige oder achteckige Form aufweist. Die eckige Form der Öffnung und der Gelenkachse erleichtert beispielsweise ein Ausrichten des ersten und zweiten Gelenkteils zueinander vor dem Zusammenfügen.

Weiterhin kann an dem erfindungsgemäßen teilbaren Orthesengelenk ein einstellbarer Anschlag zur Einstellung eines Bewegungsbereichs des Orthesengelenks vorgesehen sein. In diesem Fall kann der einstellbare Anschlag ein am ersten Gelenkteil dem zweiten Gelenkteil zugewandt befestigtes Führungselement zur Begrenzung des Bewegungsbereichs des zweiten Gelenkteils relativ zum ersten Gelenkteil aufweisen. Der Bewegungsbereich kann dann vorteilhafterweise mittels am Führungselement angeordneten Schrauben oder Madenschrauben einstellbar sein. Weiterhin ist es vorteilhaft, wenn der Anschlag mit Anschlagdämpfern ausgestattet ist. Eine Härte des Anschlags kann vorzugsweise variierbar sein. Als Anschlagdämpfer kann der Anschlag Gummipuffer aufweisen, welche am Führungselement bzw. an den Schrauben oder Madenschrauben befestigt sind. Die Härte des Anschlags kann dann durch Auswahl einer Härte der Gummipuffer eingestellt werden.

In einer weiteren vorteilhaften Ausgestaltung der Erfindung kann das teilbare Orthesengelenk eine Rückstelleinrichtung aufweisen, welche mit dem ersten und dem zweiten Gelenkteil verbunden ist und eingerichtet ist, eine Rückstellkraft zwischen dem ersten und dem zweiten Gelenkteil und somit zwischen dem ersten und dem zweiten Schalenteil aufzubringen. Dabei ist es vorteilhaft, wenn die Größe der Rückstellkraft variierbar ist. Die Rückstelleinrichtung kann beispielsweise einen Federstab und ein Halteelement für den Federstab umfassen, wobei das Halteelement am ersten Gelenkteil befestigt ist. Der Federstab kann ferner mit dem zweiten Gelenkteil form- und/oder kraftschlüssig verbunden sein. Durch eine relative Verdrehung des ersten und zweiten Gelenkteils zueinander kann der Federstab verbogen werden. Der Federstab wirkt dann dem Verbogenwerden mit der Rückstellkraft entgegen. Im Falle eines Federstabs kann die Größe der Rückstellkraft durch Variation einer Federstärke und einer Länge des Federstabes variiert werden. Alternativ zu einem Federstab kann das teilbare Orthesengelenk auch eine Zugfeder, eine Torsionsfeder, einen Gummizug, eine Gasdruckfeder, eine Gaszugfeder oder ähnliche Element als Rückstelleinrichtung aufweisen.

Die vorliegende Erfindung schließt weiterhin eine Orthese zur Korrektur einer Gelenkfehlstellung sowie Lagerung und Stabilisation eines Gelenks mit einem vorstehend beschriebenen teilbaren Orthesengelenk ein. Die erfindungsgemäße Orthese umfasst weiterhin einen ersten Schalenteil und einen zweiten Schalenteil, wobei der erste Gelenkteil am ersten Schalenteil fixiert ist und der zweite Gelenkteil am zweiten Schalenteil fixiert ist. Die Schalenteile der Orthese werden vorzugsweise mittels eines 3D-Druckverfahrens oder eines Gießverfahrens hergestellt. Die Gelenkteile können insbesondere an die Schalenteile angeschraubt, angenietet und/oder mit den Schalenteilen verklebt sein, wodurch auf ein Einlaminieren der Gelenkteile verzichtet werden kann. Dies verbessert die Steifigkeit und Stabilität der Orthese. Ferner können die Schalenteile erst fertig hergestellt und anschließend die Gelenkteile mittels Schrauben, Nieten und/oder Verkleben an den fertigen Schalenteilen befestigt werden. Alternativ können die Gelenkteile jedoch auch in die Schalenteile einlaminiert oder eingegossen sein.

Die erfindungsgemäße Orthese kann eine Bein-, Unterschenkel-, Arm- oder Unterarmorthese sein.

Im Folgenden werden einige Beispiele einer erfindungsgemäßen Orthese beschrieben. Die gezeigten einzelnen Merkmale eines Beispiels können dabei auch unabhängig vom konkreten Beispiel und in beliebiger Kombination die vorliegende Erfindung weiterbilden.

In der nachfolgenden Beschreibung der Figuren bezeichnen gleiche oder ähnliche Bezugszeichen gleiche oder ähnliche Elemente. Es zeigen
- Figur 1: ein erstes Ausführungsbeispiel eines erfindungsgemäßen teilbaren Orthesengelenks in einer Explosionsdarstellung,
- Figur 2: das Orthesengelenk aus Figur 1 in geöffnetem Zustand in einer Teilansicht,
- Figur 3: das Orthesengelenk aus Figur 1 in geschlossenem Zustand in einer Teilansicht,
- Figur 4: das Orthesengelenk aus Figur 1 in einer Teilansicht mit Schnitt senkrecht zur Gelenkachse durch den zweiten Gelenkteil,
- Figur 5: das Orthesengelenk aus Figur 1 in einer Teilansicht mit Schnitt senkrecht zur Gelenkachse durch den zweiten Gelenkteil,
- Figur 6: eine erfindungsgemäße Orthese mit dem Orthesengelenk aus Figur 1 in einer Seitenansicht,
- Figur 7: die Orthese aus Figur 6 in einer Vorderansicht,
- Figur 8: ein zweites Ausführungsbeispiel eines erfindungsgemäßen Orthesengelenks in einer Explosionsdarstellung,
- Figur 9: das Orthesengelenk aus Figur 8 n geöffnetem Zustand in einer Seitenansicht,
- Figur 10: das Orthesengelenk aus Figur 8 in geschlossenem Zustand in einer Seitenansicht,
- Figur 11: das Orthesengelenk aus Figur 8 in Schnittansicht senkrecht zur Gelenkachse durch den zweiten Gelenkteil,
- Figur 12: das Orthesengelenk aus Figur 11 in einer Schnittansicht senkrecht zur Gelenkachse durch den zweiten Gelenkteil,
- Figur 13: ein drittes Ausführungsbeispiel eines erfindungsgemäßen teilbaren Orthesengelenks in einer Explosionsdarstellung.

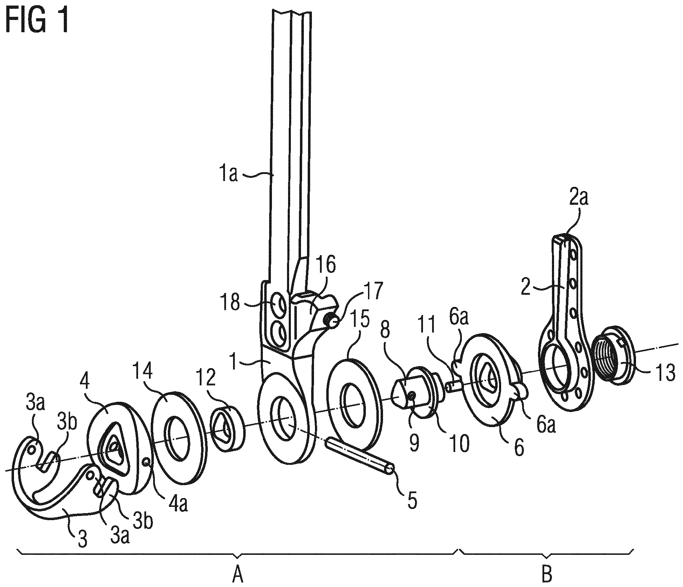

Figur 1 zeigt ein erfindungsgemäßes teilbares Orthesengelenk in einer Explosionsdarstellung. Das Orthesengelenk gliedert sich im Wesentlichen in einenTeil A und einen Teil B, welche mittels der Verschlussvorrichtung 3, 4, 5, 6 werkzeugfrei voneinander trennbar und zusammenfügbar sind. Teil A weist dabei einen ersten Gelenkteil 1, 1a auf, welcher über eine Gelenkschiene 1a an einem ersten Schalenteil einer Orthese angeschraubt werden kann. Teil B umfasst einen zweiten Gelenkteil 2, welcher an einem zweiten Schalenteil der Orthese befestigt werden kann. Sind der erste und der zweite Gelenkteil 1 und 2 voneinander getrennt, befindet sich das Orthesengelenk in einem geöffneten Zustand. Sind der erste und der zweite Gelenkteil 1 und 2 zusammengefügt, befindet sich das Orthesengelenk in einem geschlossenen Zustand.

### Zunächst wird Teil A beschrieben:

Der erste Gelenkteil 1 ist im Wesentlichen ringförmig mit einer rechteckigen Ausbuchtung ausgebildet. Diese Ausbuchtung dient als Aufnahme für die Gelenkschiene 1a, welche mittels Schrauben 18 an einer dem Teil B abgewandten Seite an der Ausbuchtung des ersten Gelenkteils 1 befestigt ist. Innerhalb des Rings des ersten Gelenkteils 1 ist ein ringförmiges Drehlager 12 des Orthesengelenks konzentrisch angeordnet und weist eine dreieckige Ringöffnung auf. Das Drehlager weist bevorzugt Teflon für eine gute Leichtgängigkeit auf. Durch die dreieckige Ringöffnung des Drehlagers 12 verläuft die Gelenkachse 8, welche einen der dreieckigen Ringöffnung entsprechenden dreieckigen Querschnitt aufweist. Beidseits des ersten Gelenkteils 1 sind, bezogen auf eine Richtung der Gelenkachse 8, ringförmige Zwischenscheiben 14 und 15 angeordnet. Die Ringöffnungen der Zwischenscheiben 14 und 15 weisen in etwa den gleichen Durchmesser wie das Drehlager 12 auf. Die Breite des Drehlagers 12, bzw. die Höhe des zylinderförmigen Drehlagers 12, ist so bemessen, dass zusätzlich zum ersten Gelenkteil 1 auch die Zwischenscheiben 14 und 15 auf dem Drehlager 12 gelagert sind.

Auf der dem Teil B, und dem zweiten Gelenkteil 2, abgewandten Außenseite des Gelenks schließt sich ein Klemmelement 3, 4, 5 an. Das Klemmelement 3, 4, 5 weist ein Grundelement 4, eine im Grundelement 4 gelagerte Bügelachse 5 und einen über die Bügelachse 5 mit dem Grundelement 4 drehbar verbundenen Klemmbügel 3 auf. Das Grundelement 4 ist ringförmig ausgebildet mit einer ebenfalls dreieckigen Ringöffnung. Das Grundelement 4 ist direkt im Anschluss an die auf der dem zweiten Gelenteil 2 abgewandten Außenseite des ersten Gelenkteils 1 angeordnete Zwischenscheibe 14 auf die dreieckige Gelenkachse 8 aufgeschoben. Das Grundelement 4 ist weiterhin im Bereich der Gelenkachse 8 verbreitert, weist also in einer Schnittebene parallel zur Gelenkachse 8 und zu einer Längsrichtung der Gelenkschiene 1a einen linsenförmigen Querschnitt auf. Der breiteste bzw. dickste Bereich des Grundelements 4 befindet sich im Bereich der Gelenkachse 8. In diesem Bereich weist das Grundelement 4 in einer Richtung senkrecht zur Längsrichtung der Gelenkschiene 1a und zur Gelenkachse 8 Öffnungen 4a auf, durch welche die Bügelachse 5 verläuft. Die Bügelachse 5 verläuft auch direkt durch die dreieckige Gelenkachse 8. Hierzu weist die Gelenkachse 8 eine Öffnung 9 auf. Über eine entlang der Mittelachse der Gelenkachse 8 eingeschraubte und gegen die Bügelachse 5 drückende Madenschraube 11 wird die Bügelachse 5 in den Öffnungen 4a und 9 gesichert. Die Bügelachse 5 steht zu beiden Seiten senkrecht zur Gelenkachse 8 aus dem Grundelement 4 heraus. An diesen überstehenden Enden der Bügelachse 5 ist der Klemmbügel 3 um die Bügelachse 5 drehbar aufgehängt. Hierzu weist der halbkreisförmig gebogene Klemmbügel 3 zwei gabelartige Enden mit jeweils zwei Gabelästen 3a und 3b auf. Der Klemmbügel 3 ist an den im geschlossenen Zustand oberen, also an den der Gelenkschiene 1a zugewandten, Gabelästen 3a an der Bügelachse 5 befestigt. Mit den unteren Gabelästen 3b kann der Klemmbügel 3 mit einem Gegenklemmelement 6 des Teils B verklemmt werden.

Auf der dem Teil B zugewandten Seite ist an einem dem zweiten Gelenkteil 2 zugewandten Ende der Gelenkachse 8 eine kreisscheibenförmige Abschlussscheibe 10 auf der Gelenkachse 8 befestigt. Der Durchmesser der Abschlussscheibe 10 ist größer als der Durchmesser der Ringöffnungen der Zwischenscheiben 14 und 15 und des ersten Gelenkteils 1 und verhindert somit ein Abrutschen der auf die Gelenkachse 8 aufgeschobenen Komponenten, also des Klemmteiles 3, 4, 5, der Zwischenscheiben 14 und 15, des Drehlagers 12 und des ersten Gelenkteils 1, wenn das Orthesengelenk geöffnet wird, also Teil A und B voneinander getrennt werden. Ferner werden somit durch die Abschlussscheibe 10 auf der dem zweiten Gelenkteil 2 zugewandten Seite und die Bügelachse 5 auf der dem zweiten Gelenkteil 2 abgewandten Seite der Gelenkachse 8 die auf die Gelenkachse 8 aufgeschobenen Komponenten des Teils A des Orthesengelenks zusammengepresst und auf der Gelenkachse 8 fixiert.

Der erste Gelenkteil 1 weist auf seiner dem zweiten Gelenkteil 2 zugewandten Seite im Bereich der Schrauben 18 ein Führungselement 16 für einen mittels Madenschrauben 17 einstellbaren Anschlag auf. Die Madenschrauben 17 lassen sich zur Gelenkschiene 1a hin einschrauben. Hierdurch kann ein Bewegungsbereich des ersten und zweiten Gelenkteils 1, 2 relativ zueinander begrenzt werden, durch Herausschrauben der Madenschrauben 17 vergrößert werden und/oder jeweils verschoben werden.

### Als nächstes wird Teil B des Orthesengelenks beschrieben:

Teil B des Orthesengelenks weist den zweiten Gelenkteil 2 auf. Der zweite Gelenkteil 2 ist im Bereich der Gelenkachse 8 ringförmig ausgebildet und nach oben hin, also in eine Erstreckungsrichtung der Gelenkschiene 1a, schienenartig verlängert, sodass der zweite Gelenkteil 2 im geschlossenen Zustand des Orthesengelenks über das Führungselement 16 hinausragt. Auf einer dem ersten Gelenkteil 1 zugewandten Außenseite des zweiten Gelenkteils 2 weist der zweite Gelenkteil 2 um seine Ringöffnungen herum eine Wandung auf, die als schmaler Mittelsteg 2a bis in den schienenartigen Bereich weitergeführt ist. Im geschlossenen Zustand des Orthesengelenks befindet sich der Mittelsteg zwischen den Madenschrauben 17 innerhalb des Führungselements 16 des ersten Gelenkteils 1 und kann die Bewegungsbeschränkung durch die Madenschrauben 17 aufnehmen.

Auf einer dem ersten Gelenkteil 1 abgewandten Innenseite des zweiten Gelenkteils weist Teil B des Orthesengelenks ein ringförmiges Befestigungselement 13 auf, wobei in einer Innenwandung des Befestigungselements 13 ein Gewinde eingebracht ist.

Auf der Außenseite des zweiten Gelenkteils 2 ist ein Gegenklemmelement 6 angeordnet. Das Gegenklemmelement 6 weist zum ersten Gelenkteil 1 hin eine Ringscheibe auf, welche auf Höhe der Gelenkachse 8, bezogen auf eine Erstreckungsrichtung der Gelenkschiene 1a diametral angeordnete Klemmnasen 6a aufweist, welche mit den Gabelästen 3b des Klemmbügels 3 im geschlossenen Zustand des Orthesengelenks verklemmt werden. Die Ringscheibe des Gegenklemmelements 6 weist eine Ringöffnung mit einer Dicke und einem Durchmesser auf, der einer Dicke und dem Durchmesser der Abschlussscheibe 10 entspricht, sodass im geschlossenen Zustand des Orthesengelenks die Abschlussscheibe 10 vollständig von der Ringöffnung des Gegenklemmelements 6 aufgenommen werden kann. Auf einer dem zweiten Gelenkteil 2 zugewandten Innenseite des Gegenklemmelements 6 ist ein weiteres ringförmiges, integral mit dem Gegenklemmelement 6 ausgebildetes Element angeordnet. Dieses Element weist eine Ringöffnung mit dreieckigem Querschnitt auf, welcher dem Querschnitt der Gelenkachse 8 entspricht. Auf einer der Ringöffnung abgewandten Außenwandung des Elements ist ein Gegengewinde zum Gewinde des Befestigungselements 13 eingebracht.

Die Ringöffnung des zweiten Gelenkteils 2 ist so bemessen, dass zur Befestigung des Gegenklemmelements 6 am zweiten Gelenkteil 2 das Gewinde des Befestigungselements 13 durch die Ringöffnung des zweiten Gelenkteils 2 geschoben und das Gegenklemmelement 6 mit dem Befestigungselement 13 in der Ringöffnung des zweiten Gelenkteils 2 verschraubt werden kann.

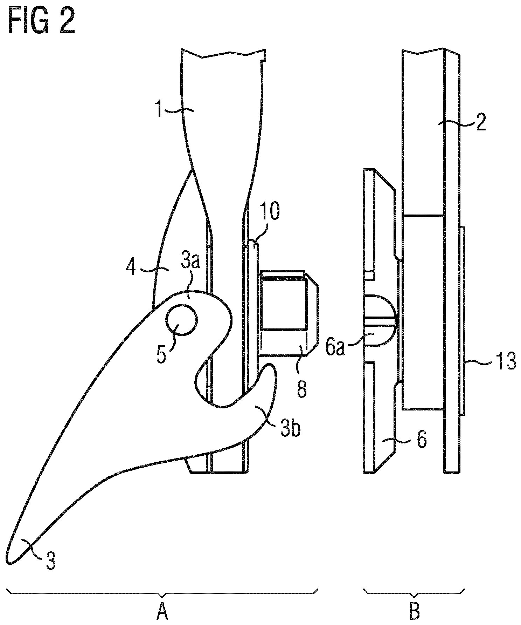

Figur 2 zeigt einen Teilbereich des Orthesengelenks aus Figur 1 in geöffnetem Zustand. Die Gelenkachse 8 ist fest mit dem ersten Gelenkteil 1 verbunden und ragt zusammen mit der Abschlussscheibe 10 zum Einfügen in das Gegenklemmelement 6 heraus. Der Klemmbügel 3 ist frei beweglich um die Bügelachse 5, da die Gabeläste 3b nicht mit den Klemmnasen 6a klemmen.

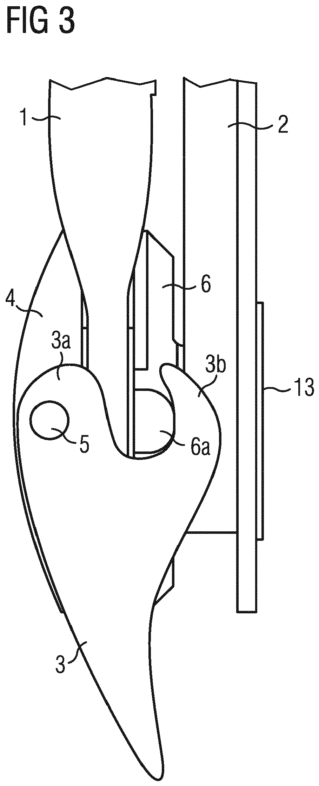

Figur 3 zeigt einen Teilbereich des Orthesengelenks aus Figur 1 in geschlossenem Zustand. Die Abschlussscheibe 10 sowie der in Figur 2 herausragende Teil der Gelenkachse 8 sind vollständig vom Gegenklemmelement 6 aufgenommen. Der Klemmbügel 3 ist fest eingerastet, da die Gabeläste 3b mit den Klemmnasen 6a klemmen.

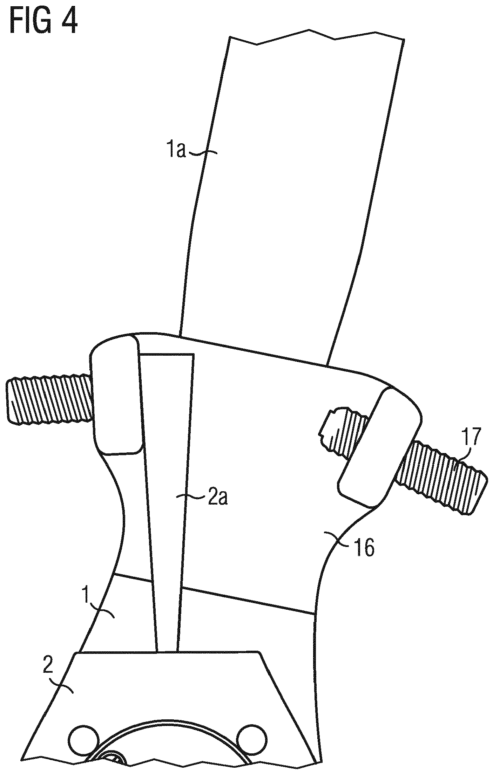

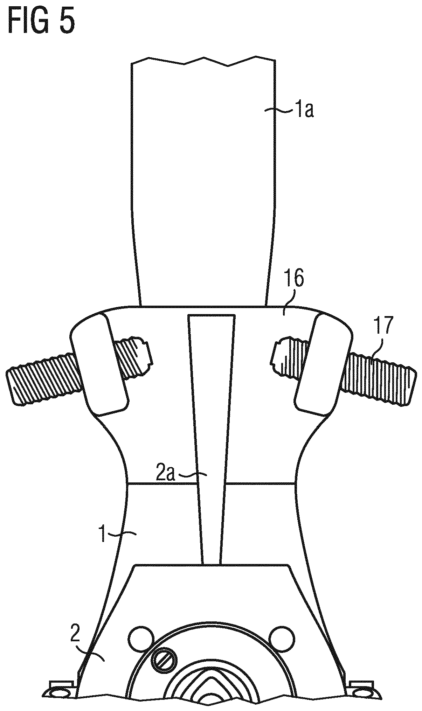

Figuren 4 und 5 zeigen die Einstellung des Bewegungsbereichs des Orthesengelenks in einer Detailansicht. Figur 4 zeigt, wie sich der Bewegungsbereich des Orthesengelenks in zueinander entgegengesetzte Richtungen verschieben lässt. Ist die Madenschraube 17 an einer ersten Seite herausgeschraubt und an der gegenüberliegenden zweiten Seite herein geschraubt, ist lediglich eine Bewegung im Winkelbereich der zweiten Seite beschränkt. Der Bewegungsbereich ist somit zur ersten Seite hin verschoben und gleichzeitig gegenüber eines maximal möglichen Bewegungsbereichs, wenn beide Madenschrauben 17 herausgeschraubt wären, eingeschränkt. Figur 5 zeigt lediglich eine Einschränkung des Bewegungsbereichs zu gleichen Teilen auf beiden Seiten, da beide Madenschrauben 17 gleich weit herein geschraubt sind.

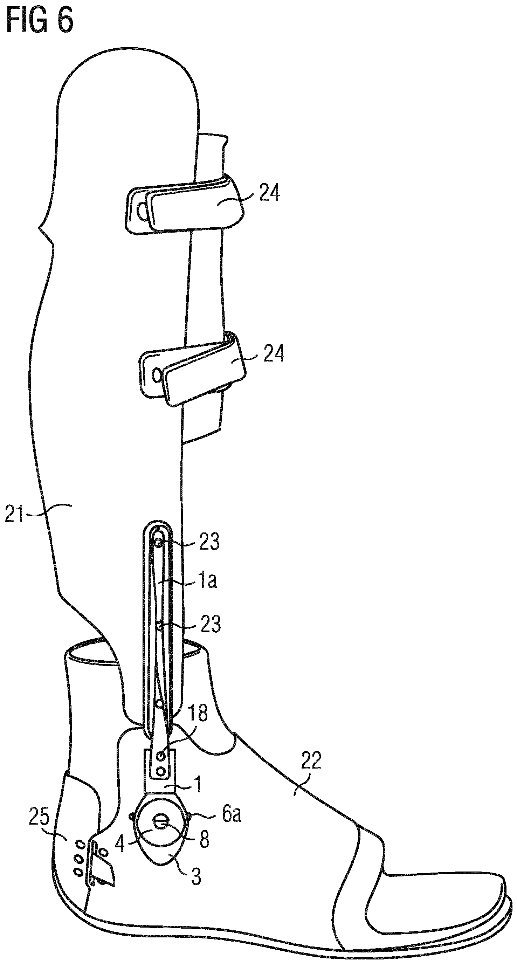

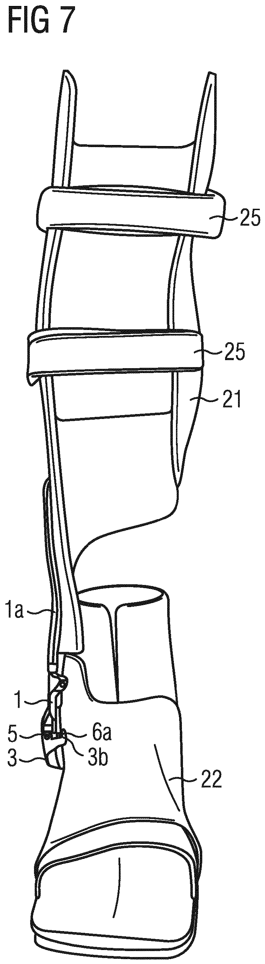

Figuren 6 und 7 zeigen eine erfindungsgemäße Unterschenkelorthese zur Korrektur einer Gelenkfehlstellung sowie zur Lagerung und Stabilisation eines Gelenks mit einem erfindungsgemäßen Orthesengelenk in geschlossenem Zustand, wobei Figur 6 eine Seitenansicht und Figur 7 eine Vorderansicht der Orthese zeigt. Die Orthese weist ein erstes Schalenteil 21 zur Aufnahme eines Unterschenkels und zweites Schalenteil 22 zur Aufnahme eines Fußes auf. Das erste Schalenteil weist in einem Schienbeinbereich zwei übereinander angeordnete Schnallen 24 zum Öffnen und Verschließen des ersten Schalenteils 21 auf. Das zweite Schalenteil 22 ist als Ringfassung mit offenem Zehenbereich und einer einstellbaren sowie öffenbaren und verschließbaren Fersenklappe 25 ausgestattet. Das Orthesengelenk ist an einer Außenseite der Orthese im Bereich des Sprunggelenks angeordnet und verbindet das erste 21 mit dem zweiten Schalenteil 22. Der erste Gelenkteil 1, welcher über Schrauben 18 mit der Gelenkschiene 1a verbunden ist, ist über die Gelenkschiene 1a im dem zweiten Schalenteil zugewandten unteren Bereich des ersten Schalenteils 21 am ersten Schalenteil 21 mittels Schrauben 23 angeschraubt. Der zweite Gelenkteil 2 (hier nicht sichtbar) ist zusammen mit dem Befestigungselement 13 (ebenfalls hier nicht sichtbar) von einem Innenbereich des zweiten Schalenteils 22 aus mittels des Gegenklemmelements 6 an das zweite Schalenteil 22 geschraubt. Im Bereich des zweiten Gelenkteils 2 weist das zweite Schalenteil 22 eine Aussparung auf (hier nicht sichtbar), damit der Mittelsteg 2a des zweiten Gelenkteils 2 im Führungselement des ersten Gelenkteils 1 geführt werden kann.

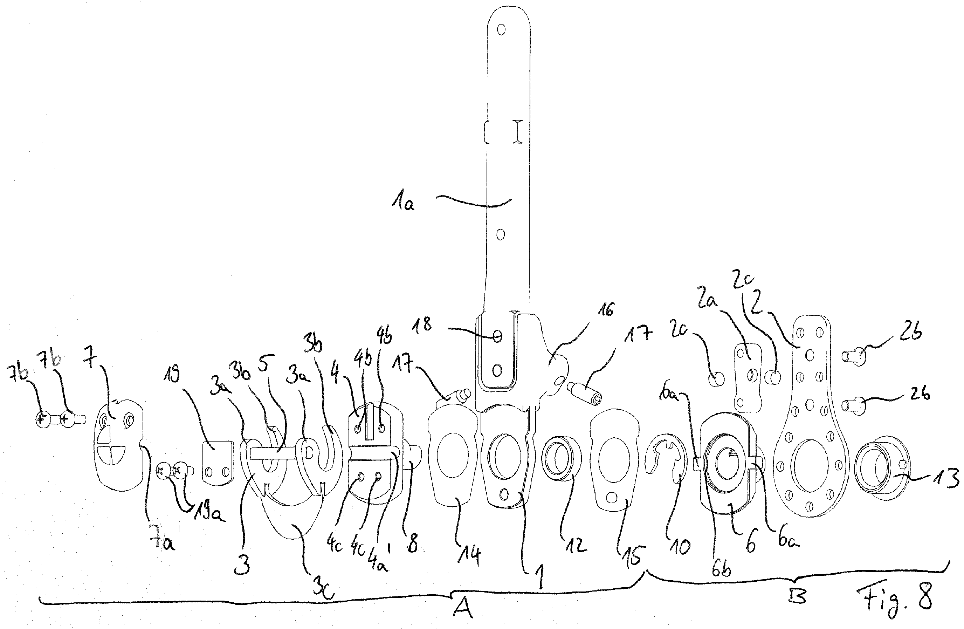

Figur 8 zeigt ein zweites Ausführungsbeispiel eines erfindungsgemäßen teilbaren Orthesengelenks in einer Explosionsdarstellung. Wie im ersten Ausführungsbeispiel gliedert sich das teilbare Orthesengelenk gemäß dem zweiten Ausführungsbeispiel in einen Teil A und einen Teil B, welche mittels einer Verschlussvorrichtung 3, 4, 5, 6 werkzeugfrei voneinander trennbar und zusammenfügbar sind. Im Unterschied zum ersten Ausführungsbeispiel weist der zweite Gelenkteil 2 keinen Mittelsteg, sondern ein Anschlagselement 2a auf, welches mittels Schrauben 2b am zweiten Gelenkteil 2 befestigt ist. Das Anschlagelement 2a verfügt über zwei Gummipuffer 2c, welche seitlich am Anschlagelement 2a angeordnet sind, sodass die Gummipuffer 2c im geschlossenen Zustand des Orthesengelenks den Madenschrauben 17 zugewandt sind. Die Gummipuffer 2c dienen als Anschlagdämpfer, welche das Auftreffen der einstellbaren Madenschrauben 17 auf das Anschlagelement 2a dämpfen. Durch Wahl einer bestimmten Gummihärte kann die Härte des Anschlags eingestellt werden.

Ein weiterer wesentlicher Unterschied zum ersten Ausführungsbeispiel bezieht sich auf die Ausgestaltung des Grundelements 4 und der Lagerung der Bügelachse 5. In Figur 8 ist das Grundelement in einen Teil 4 und einen Teil 7 getrennt, welche mittels der Schrauben 7b und der Gewindeöffnungen 4b miteinander verschraubt sind. Die Bügelachse 5 ist in einer zusammen vom Teil 4 und Teil 7 gebildeten Aussparung 7a und 4a'gelagert. Die Bügelachse 5 ist weiterhin nicht vollständig zylinderförmig ausgebildet, sondern weist einen quer zur Längsachse verlaufenden, abgeflachten Querschnitt auf. Die Durchgangsöffnungen in den Gabelästen 3a zur Aufnahme der Bügelachse 5 weisen ebenso einen kreisförmigen Querschnitt mit einer abgeflachten Seite auf. Durch die Abflachung wird die Bügelachse 5 in den Durchgangsöffnungen der Gabeläste 3a verdrehsicher gehalten. Die abgeflachte Seite der Bügelachse 5 ist im geschlossenen Zustand des Orthesengelenks vom zweiten Gelenkteil weg weisend ausgerichtet. Unterhalb, also auf einer von der Gelenkschiene 1a abgewandten Seite der Bügelachse 5, ist eine Blattfeder 19 mittels Schrauben 19a am Teil 4 des Grundelements befestigt. Die Blattfeder 19 ist derart positioniert, dass sie gegen die abgeflachte Seite der Bügelachse drückt. Durch die Blattfeder rastet der Klemmbügel 3 im geschlossenen Zustand ein und wird verriegelt gehalten. Mittels eines Bedienhebels 3c kann der Klemmbügel 3c ferner leicht geöffnet und geschlossen werden.

Ein weiterer Unterschied zum ersten Ausführungsbeispiel besteht in der Ausbildung und Halterung der Abschlussscheibe 10 sowie der Gelenkachse 8. Die Gelenkachse 8 weist einen grob kreisförmigen Querschnitt mit einem unregelmäßig ausgebildeten Rand auf. Die Abschlussscheibe 10 ist als offener Klemmring ausgestaltet. Die Abschlussscheibe 10 klemmt dabei in einer Nut 6b, welche um die Durchgangsöffnung des Gegenklemmelements 6 für die Gelenkachse 8 ausgebildet ist. Im eingeklemmten Zustand ist die als Klemmring ausgebildete Abschlussscheibe nahezu vollständig oder vollständig geschlossen. Durch das Verklemmen in der Nut 6b wird ein Verdrehen der Abschlussscheibe 10 gegenüber dem Gegenklemmelement 6 und somit auch gegenüber dem zweiten Gelenkteil 2 verhindert. Im eingeklemmten Zustand weist die Abschlussscheibe 10 ebenfalls eine grob kreisförmige Durchgangsöffnung mit einem unregelmäßig geformten Rand auf. Durch die unregelmäßige Form des Randes des Achsquerschnitts und der Durchgangsöffnung der Abschlussscheibe 10 wird ein Verdrehen der Achse 8 relativ zum zweiten Gelenkteil 2 und somit zum Teil B verhindert. Somit ist auch in diesem Ausführungsbeispiel der zweite Gelenkteil 2 starr mit der Gelenkachse 8 verbunden, während der erste Gelenkteil 1 um die Gelenkachse 8 drehbar auf dem Lager 12 angeordnet ist.

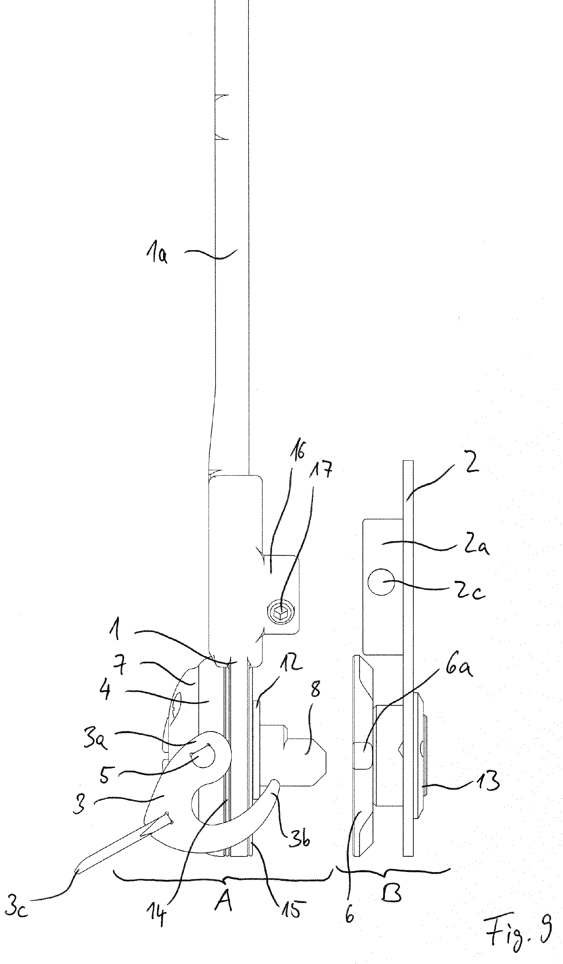

Figur 9 zeigt das Orthesengelenk aus Figur 8 in geöffnetem Zustand. Die Gelenkachse 8 ist in Richtung der Gelenkachse 8 fest mit dem ersten Gelenkteil 1 verbunden, wobei der erste Gelenkteil jedoch um die erste Gelenkachse 8 drehbar ist, und ragt zusammen mit einem Bereich des Lagers 12 zum Einfügen in das Gegenklemmelement 6 aus dem ersten Gelenkteil heraus. Der Klemmbügel 3 ist fest mit der Bügelachse 5 verbunden, wobei der Klemmbügel 3 zusammen mit der Bügelachse 5 innerhalb der Aussparung 4a', 7a des zusammengesetzten Grundelements 4, 7 frei beweglich ist, da die Gabeläste 3b nicht mit den Klemmnasen 6a klemmen.

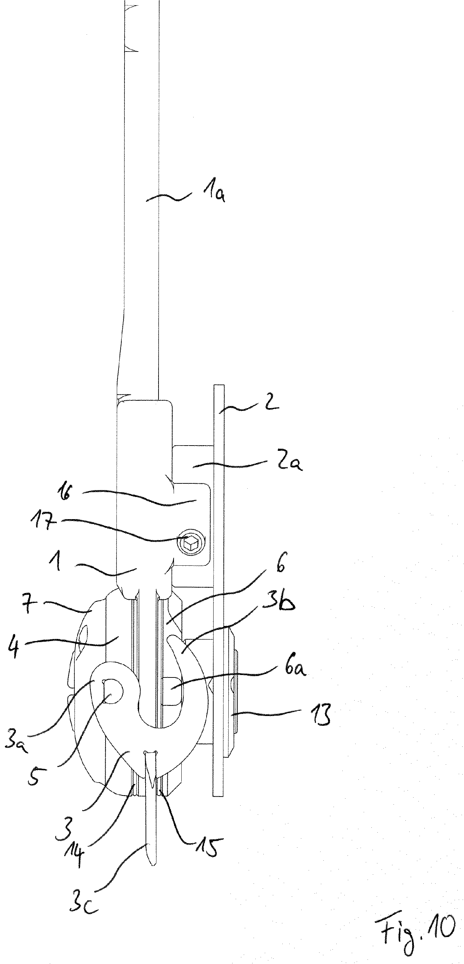

Figur 10 zeigt das Orthesengelenks aus Figur 8 in geschlossenem Zustand. Das Lager 12 sowie der in Figur 9 herausragende Teil der Gelenkachse 8 sind vollständig vom Gegenklemmelement 6 aufgenommen. Der Klemmbügel 3 ist fest eingerastet, sodass die Gabeläste 3b mit den Klemmnasen 6a klemmen, und wird von der hier nicht sichtbaren Blattfeder 19 verriegelt gehalten..

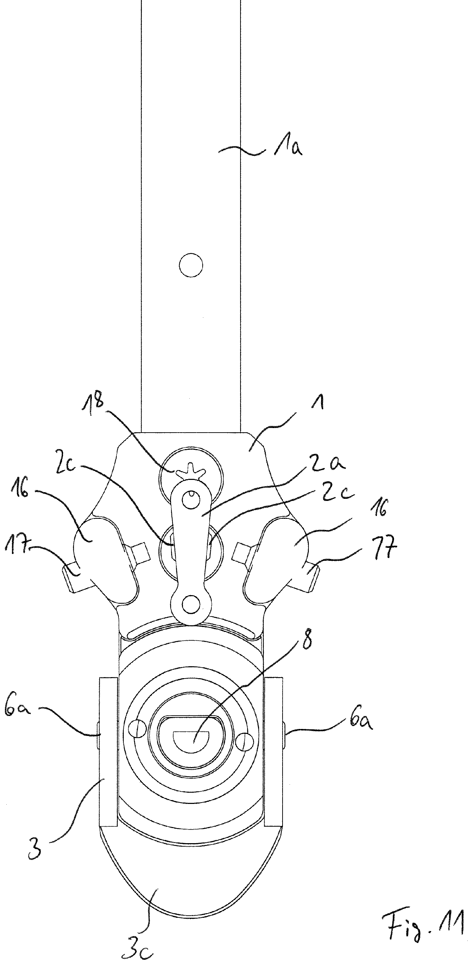

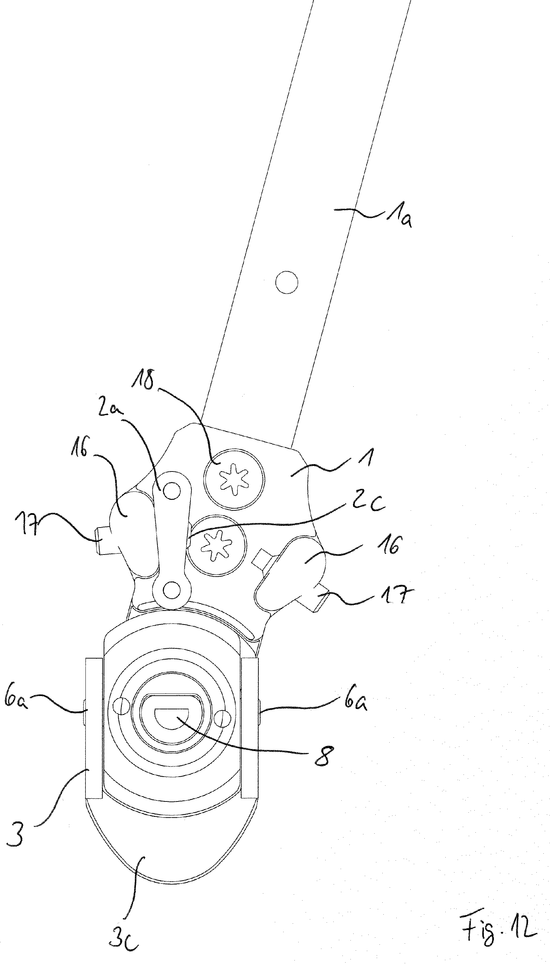

Figuren 11 und 12 zeigen den durch die Führungselemente und Madenschrauben 17 beschränkten Bewegungsbereich des Orthesengelenks sowie das Anschlagelement 2a in einer Schnittansicht senkrecht zur Gelenkachse 8 durch den zweiten Gelenkteil 2. In Figur 11 befindet sich das Anschlagelement 2a in mittlerer Position, das heißt, das Orthesengelenk befindet sich in einer geraden Nullstellung. Beidseits des Anschlagelements 2a sind jeweils den Madenschrauben 17 zugewandt als Anschlagdämpfer ausgebildete Gummipuffer 2c angeordnet. Figur 12 zeigt, dass diese Gummipuffer 2c gegen die Madenschrauben 17 stoßen, wenn der erste Gelenkteil 1 ein Ende des eingestellten Bewegungsbereichs erreicht. Die Gummipuffer 2c dämpfen dabei den Stoß des Anschlagelements 2a mit dem Führungselement 16 ab.

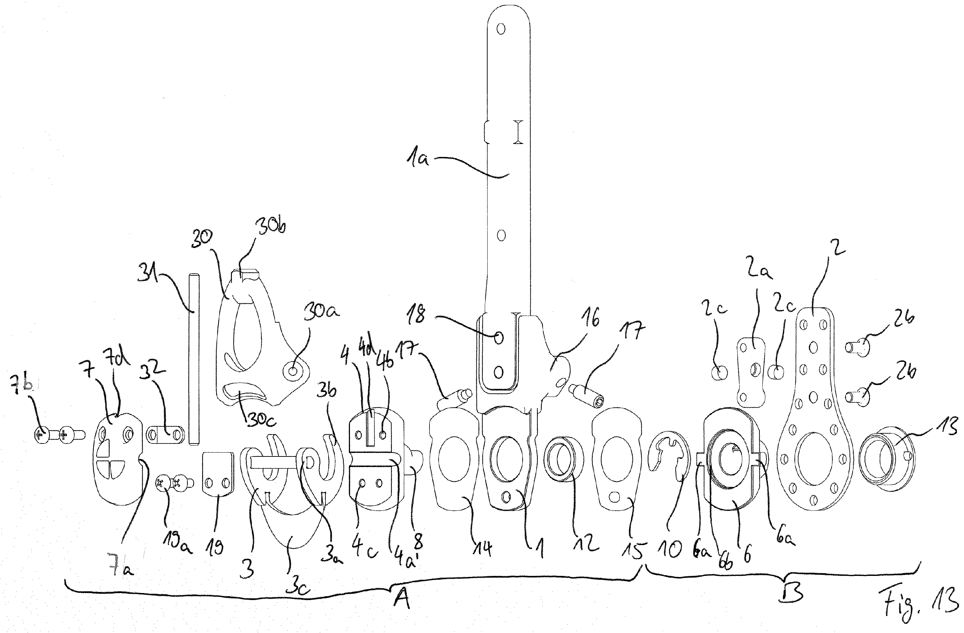

Figur 13 zeigt ein drittes Ausführungsbeispiel eines erfindungsgemäßen teilbaren Orthesengelenks in einer Explosionsdarstellung. Im Unterschied zum zweiten Ausführungsbeispiel weist das Orthesengelenk in Figur 13 eine Rückstelleinrichtung 30, 31 auf. Die Rückstelleinrichtung 30, 31 weist ein Halteelement 30 und einen Federstab 31 auf. Das Halteelement 30 ist im Wesentlichen ringförmig ausgebildet und über Befestigungspunkte 30a am Führungselement 16 mittels der Madenschrauben 17 und somit am ersten Gelenkteil 1 befestigt. Das Halteelement 30 weist ferner einer Richtung senkrecht zur Gelenkachse 8 zwei Durchgangsöffnungen 30b und 30c auf. Durch diese Durchgangsöffnungen 30b und 30c verläuft der Federstab 31. Dabei verläuft der Federstab 31 auch in einer von dem zusammengesetzten Grundelement 4, 7 gebildeten Aussparung, welche senkrecht zur Gelenkachse 8 verläuft. Der Federstab ist zusätzlich mittels einer Schelle 32 im Grundelement 4, 7 mittels der Schrauben 7b fixiert. Durch eine relative Verdrehung des ersten 1 und zweiten Gelenkteils 2 zueinander wird der Federstab 31 verbogen. Der Federstab 31 übt dann eine Rückstellkraft gegen das Verbiegen auf die Gelenkteile 1 und 2 aus.

## Patentansprüche

1. Teilbares Orthesengelenk (A,B) für eine bewegliche Verbindung zweier Schalenteile (21,22) einer Orthese zur Korrektur einer Gelenkfehlstellung mit einem ersten Gelenkteil (1), der an einem ersten Schalenteil (21) fixierbar ist, und einem relativ zum ersten Gelenkteil (1) um eine Gelenkachse (8) des Orthesengelenks drehbaren zweiten Gelenkteil (2), der an einem zweiten Schalenteil (22) fixierbar ist,
**gekennzeichnet durch**
eine Verschlussvorrichtung (3,4,5,6) zum werkzeugfreien Trennen und Zusammenfügen des ersten und zweiten Gelenkteils (1,2).

2. Teilbares Orthesengelenk (A,B) nach dem vorhergehenden Anspruch, wobei die Verschlussvorrichtung (3,4,5,6) zum werkzeugfreien Trennen und Zusammenfügen des ersten und zweiten Gelenkteils (1,2) an der Gelenkachse (8) ausgebildet ist.

3. Teilbares Orthesengelenk (A,B) nach dem vorhergehenden Anspruch, wobei die Verschlussvorrichtung (3,4,5,6) ein mit dem ersten Gelenkteil (1) verbundenes Klemmelement (3,4,5) und ein am zweiten Gelenkteil (2) befestigtes Gegenklemmelement (6) aufweist, sodass zum Zusammenfügen des ersten und zweiten Gelenkteils (1,2) das Klemmelement (3,4,5) mit dem Gegenklemmelement (6) in Richtung der Gelenkachse (8) verklemmbar ist.

4. Teilbares Orthesengelenk (A,B) nach dem vorhergehenden Anspruch, wobei zum Zusammenfügen des ersten und zweiten Gelenkteils (1,2) der erste Gelenkteil (1) zwischen dem Klemmelement (3,4,5) und dem Gegenklemmelement (6) positionierbar ist und der zweite Gelenkteil (2) auf einer dem ersten Gelenkteil (1) abgewandten Seite des Gegenklemmelements (6) positionierbar ist, bezogen auf eine Richtung der Gelenkachse (8).

5. Teilbares Orthesengelenk (A,B) nach einem der Ansprüche 3 oder 4, wobei das Klemmelement (3,4,5) ein Grundelement (3), eine in dem Grundelement (4) angeordnete, senkrecht zur Gelenkachse (8) verlaufende Bügelachse (5) und einen an der Bügelachse (5) drehbar aufgehängten Klemmbügel (3) aufweist, wobei der Klemmbügel (3) durch Drehen um die Bügelachse (5) mit dem Gegenklemmelement (6) verklemmbar und lösbar ist.

6. Teilbares Orthesengelenk (A,B) nach einem der zwei vorhergehenden Ansprüche, wobei der Klemmbügel (3) zwei gabelartige Enden aufweist und an jeweils einem ersten Ast (3a) der gabelartigen Enden drehbar an der Bügelachse (5) aufgehängt ist und mit jeweils einem zweiten Ast (3b) der gabelartigen Enden mit dem Gegenklemmelement (6) verklemmbar ist, und wobei das Gegenklemmelement (6) zwei gegenüberliegend angeordnete Klemmnasen (6a) zum Verklemmen mit jeweils dem zweiten Ast (3b) der gabelartigen Enden des Klemmbügels (3) aufweist.

7. Teilbares Orthesengelenk (A,B) nach einem der vorhergehenden Ansprüche, wobei das Orthesengelenk ein Drehlager (12) umfasst, das einen fest mit der Gelenkachse (8) verbundenen Ring (10) aufweist, auf dem der erste Gelenkteil (1) drehbar gelagert ist.

8. Teilbares Orthesengelenk (A,B) nach einem der vorhergehenden Ansprüche, wobei die Gelenkachse (8) bis auf eine Drehbeweglichkeit des ersten Gelenkteils (1) relativ zur Gelenkachse (8) fest mit dem ersten Gelenkteil (1) verbunden ist.

9. Teilbares Orthesengelenk (A,B) nach einem der vorhergehenden Ansprüche, wobei das Gegenklemmelement (6) eine Öffnung zum Aufnehmen der Gelenkachse (8) aufweist.

10. Teilbares Orthesengelenk (A,B) nach dem vorhergehenden Anspruch, wobei die Gelenkachse (8) einen eckigen, insbesondere dreieckigen, viereckigen, fünfeckigen, sechseckigen oder achteckigen Querschnitt aufweist und die Öffnung eine entsprechend eckige, insbesondere dreieckige, viereckige, fünfeckige, sechseckige oder achteckige Form aufweist.

11. Teilbares Orthesengelenk (A,B) nach einem der vorhergehenden Ansprüche, **gekennzeichnet durch** einen einstellbaren Anschlag (16,17) zur Einstellung eines Bewegungsbereichs des Orthesengelenks.

12. Teilbares Orthesengelenk (A,B) nach dem vorhergehenden Anspruch, wobei der einstellbare Anschlag (16,17) ein am ersten Gelenkteil (1) und dem zweiten Gelenkteil (2) zugewandt befestigtes Führungselement (16) zur Begrenzung des Bewegungsbereichs des zweiten Gelenkteils (2) relativ zum ersten Gelenkteil (1) aufweist.

13. Teilbares Orthesengelenk (A,B) nach dem vorhergehenden Anspruch, wobei der Bewegungsbereich mittels am Führungselement (16) angeordneten Schrauben und/oder Madenschrauben (17) einstellbar ist.

14. Teilbares Orthesengelenk (A,B) nach einem der vorhergehenden Ansprüche, **gekennzeichnet durch** eine Rückstelleinrichtung (30,31), welche mit dem ersten und dem zweiten Gelenkteil (1,2) verbunden ist und eingerichtet ist, eine Rückstellkraft zwischen dem ersten und dem zweiten Gelenkteil (1,2) aufzubringen.

15. Orthese zur Korrektur einer Gelenkfehlstellung mit einem teilbaren Orthesengelenk (A,B) nach einem der vorhergehenden Ansprüche, einem ersten Schalenteil (21,22) und einem zweiten Schalenteil (22), wobei der erste Gelenkteil (1) am ersten Schalenteil (21) fixiert ist und der zweite Gelenkteil (2) am zweiten Schalenteil (22) fixiert ist.

## Claims

1. Divisible orthotic joint (A, B) for a movable connection of two shell parts (21, 22) of an orthosis for correcting a malpositioned joint, with a first joint part (1), which is fixable on a first shell part (21), and a second joint part (2) which is rotatable relative to the first joint part (1) about a joint axis (8) of the orthotic joint and is fixable on a second shell part (22),
**characterized by**
a closure device (3, 4, 5, 6) for separating and joining together the first and second joint parts (1, 2) without the use of tools.

2. Divisible orthotic joint (A, B) according to the preceding claim, wherein the closure device (3, 4, 5, 6) for separating and joining together the first and second joint parts (1, 2) without the use of tools is formed on the joint axis (8).

3. Divisible orthotic joint (A, B) according to the preceding claim, wherein the closure device (3, 4, 5, 6) has a clamp element (3, 4, 5) connected to the first joint part (1), and a mating clamp element (6) secured to the second joint part (2), such that, in order to join together the first and second joint parts (1, 2), the clamp element (3, 4, 5) can be clamped with the mating clamp element (6) in the direction of the joint axis (8).

4. Divisible orthotic joint (A, B) according to the preceding claim, wherein, in order to join together the first and second joint parts (1, 2), the first joint part (1) can be positioned between the clamp element (3, 4, 5) and the mating clamp element (6), and the second joint part (2) can be positioned on a side of the mating clamp element (6) directed away from the first joint part (1), with respect to a direction of the joint axis (8).

5. Divisible orthotic joint (A, B) according to either of Claims 3 and 4, wherein the clamp element (3, 4, 5) has a main element (3), a clip spindle (5) arranged in the main element (4) and extending perpendicularly with respect to the joint axis (8), and a clamping clip (3) suspended rotatably on the clip spindle (5), wherein the clamping clip (3) can be clamped onto and released from the mating clamp element (6) by rotation about the clip spindle (5).

6. Divisible orthotic joint (A, B) according to either of the two preceding claims, wherein the clamping clip (3) has two fork-like ends and is suspended rotatably on the clip spindle (5) at a respective first branch (3a) of the fork-like ends and can be clamped to the mating clamp element (6) at a respective second branch (3b) of the fork-like ends, and wherein the mating clamp element (6) has two mutually opposite clamping lugs (6a) for clamping to the respective second branch (3b) of the fork-like ends of the clamping clip (3).

7. Divisible orthotic joint (A, B) according to one of the preceding claims, wherein the orthotic joint comprises a rotary bearing (12) having a ring (10) which is connected rigidly to the joint axis (8) and on which the first joint part (1) is rotatably mounted.

8. Divisible orthotic joint (A, B) according to one of the preceding claims, wherein the joint axis (8) is connected rigidly to the first joint part (1), except for a rotational mobility of the first joint part (1) relative to the joint axis (8).

9. Divisible orthotic joint (A, B) according to one of the preceding claims, wherein the mating clamp element (6) has an opening for receiving the joint axis (8).

10. Divisible orthotic joint (A, B) according to the preceding claim, wherein the joint axis (8) has a polygonal, in particular trigonal, tetragonal, pentagonal, hexagonal or octagonal cross section, and the opening has a correspondingly polygonal, in particular trigonal, tetragonal, pentagonal, hexagonal or octagonal shape.

11. Divisible orthotic joint (A, B) according to one of the preceding claims, **characterized by** an adjustable limit stop (16, 17) for adjusting a range of movement of the orthotic joint.

12. Divisible orthotic joint (A, B) according to the preceding claim, wherein the adjustable limit stop (16, 17) has a guide element (16) which is secured on the first joint part (1) and facing the second joint part (2) and which is configured to limit the range of movement of the second joint part (2) relative to the first joint part (1).

13. Divisible orthotic joint (A, B) according to the preceding claim, wherein the range of movement is adjustable by means of screws and/or grub screws (17) arranged on the guide element (16).

14. Divisible orthotic joint (A, B) according to one of the preceding claims, **characterized by** a restoring device (30, 31) which is connected to the first joint part (1) and second joint part (2) and is configured to apply a restoring force between the first joint part (1) and second joint part (2).

15. Orthosis for correcting a malpositoned joint, with a divisible orthotic joint (A, B) according to one of the preceding claims, a first shell part (21, 22) and a second shell part (22), wherein the first joint part (1) is fixed on the first shell part (21), and the second joint part (2) is fixed on the second shell part (22).

## Revendications

1. Articulation d'orthèse divisible (A, B) pour une liaison mobile de deux parties de coques (21, 22) d'une orthèse pour la correction d'une mauvaise position d'une articulation avec une première partie d'articulation (1), qui peut être fixée à une première partie de coque (21), et une deuxième partie d'articulation (2), rotative par rapport à la première partie d'articulation (1) autour d'un axe d'articulation (8) de l'articulation d'orthèse, qui peut être fixée à une deuxième partie de coque (22), **caractérisée par**
un dispositif de fermeture (3, 4, 5, 6) pour la séparation et l'assemblage sous outil de la première et de la deuxième parties d'articulation (1, 2).

2. Articulation d'orthèse divisible (A, B) selon la revendication précédente, le dispositif de fermeture (3, 4, 5, 6) étant conçu pour la séparation et l'assemblage sans outil de la première et de la deuxième parties d'articulation (1, 2) au niveau de l'axe d'articulation (8).

3. Articulation d'orthèse divisible (A, B) selon la revendication précédente, le dispositif de fermeture (3, 4, 5, 6) comprenant un élément de blocage (3, 4, 5) relié avec la première partie d'articulation (1) et un contre-élément de blocage (6) fixé à la deuxième partie d'articulation (2), de façon à ce que, pour l'assemblage de la première et de la deuxième parties d'articulation (1, 2), l'élément de blocage (3, 4, 5) puisse être bloqué avec le contre-élément de blocage (6) en direction de l'axe de pivotement (8).

4. Articulation d'orthèse divisible (A, B) selon la revendication précédente, moyennant quoi, pour l'assemblage de la première et de la deuxième parties d'articulation (1, 2), la première partie d'articulation (1) peut être positionnée entre l'élément de blocage (3, 4, 5) et le contre-élément de blocage (6) et la deuxième partie d'articulation (2) peut être positionnée sur un côté, opposé à la première partie d'articulation (1), du contre-élément de blocage (6), par rapport à une direction de l'axe de pivotement (8).

5. Articulation d'orthèse divisible (A, B) selon l'une des revendications 3 ou 4, l'élément de blocage (3, 4, 5) comprenant un élément de base (3), un axe d'étrier (5), disposé dans l'élément de base (4), s'étendant perpendiculairement à l'axe d'articulation (8) et un étrier de blocage (3) suspendu de manière rotative au niveau de l'axe d'étrier (5), l'étrier de blocage (3) pouvant être bloqué et libéré par la rotation autour de l'axe d'étrier (5) avec le contre-élément de blocage (6).

6. Articulation d'orthèse divisible (A, B) selon l'une des revendications précédentes, l'étrier de blocage (3) comprenant deux extrémités en forme de fourches et étant suspendu de manière rotative, à une première branche (3a) des extrémités en forme de fourches, par rapport à l'axe d'étrier (5) et peut être bloqué avec une deuxième branche (3b) des extrémités en forme de fourches, avec le contre-élément de blocage (6), et le contre-élément de blocage (6) comprenant deux embouts de blocage (6a), situés l'un en face de l'autre, pour le blocage avec la deuxième branche (3b) des extrémités en forme de fourches de l'étrier de blocage (3).

7. Articulation d'orthèse divisible (A, B) selon l'une des revendications précédentes, l'articulation d'orthèse comprenant un palier rotatif (12), qui comprend une bague (10) reliée fermement avec l'axe d'articulation (8), sur laquelle la première partie d'articulation (1) est logée de manière rotative.

8. Articulation d'orthèse divisible (A, B) selon l'une des revendications précédentes, l'axe d'articulation (8) est reliée fermement avec la première partie d'articulation (1), à l'exception d'un mouvement de rotation de la première partie d'articulation (1) par rapport à l'axe d'articulation (8).

9. Articulation d'orthèse divisible (A, B) selon l'une des revendications précédentes, le contre-élément de blocage (6) comprenant une ouverture pour le logement de l'axe d'articulation (8).

10. Articulation d'orthèse divisible (A, B) selon la revendication précédente, l'axe d'articulation (8) présentant une section angulaire, plus particulièrement triangulaire, quadrangulaire, pentagonale, hexagonale ou octogonale et l'ouverture présentant une forme angulaire, plus particulièrement triangulaire, quadrangulaire, pentagonale, hexagonale ou octogonale correspondante.

11. Articulation d'orthèse divisible (A, B) selon l'une des revendications précédentes, **caractérisée par** une butée réglable (16, 17) pour le réglage d'une zone de mouvement de l'articulation d'orthèse.

12. Articulation d'orthèse divisible (A, B) selon la revendication précédente, la butée réglable (16, 17) comprenant un élément de guidage (16) fixé de manière orientée vers la première partie d'articulation (1) et la deuxième partie d'articulation (2) pour la limitation de la zone de mouvement de la deuxième partie d'articulation (2) par rapport à la première partie d'articulation (1).

13. Articulation d'orthèse divisible (A, B) selon la revendication précédente, la zone de mouvement pouvant être réglée au moyen de vis et/ou de vis sans têtes (17) disposée sur l'élément de guidage (16).

14. Articulation d'orthèse divisible (A, B) selon l'une des revendications précédentes, **caractérisée par** un dispositif de rappel (30, 31) qui est relié avec la première et la deuxième parties d'articulation (1, 2) et est conçu pour exercer une force de rappel entre la première et la deuxième parties d'articulation (1, 2).

15. Orthèse pour la correction d'une mauvaise position d'articulation avec une articulation d'orthèse divisible (A, B) selon l'une des revendications précédentes, une première partie de coque (21, 22) et une deuxième partie de coque (22), la première partie d'articulation (1) étant fixée à la première partie de coque (21) et la deuxième partie d'articulation (2) étant fixée à la deuxième partie de coque (22).
